# EUROPEAN PATENT SPECIFICATION

(11) **EP 3 966 546 B1**
(45) Date of publication and mention of the grant of the patent: **12.04.2023**
(21) Application number: 20744093.4
(22) Date of filing: 08.05.2020
(51) Int. Cl.: G01N 3/42

(54) **WAX ANALYSER**
WACHSANALYSATOR
DISPOSITIF D'ANALYSE DE CIRE

(30) Priority: 09.05.2019 PL 42987819
(43) Date of publication of application: 16.03.2022
(73) Proprietor: Uniwersytet Marii Curie-Sklodowskiej, 20-031 Lublin (PL)
(72) Inventor: PIETROW, Marek, 20-330 Lublin (PL); GAGOS , Mariusz, 20-530 Lublin (PL); WAWRYSZCZUK, Jan, 20-032 Lublin (PL)
(74) Representative: Augustyniak, Magdalena Anna
(86) International application number: PCT/IB2020/054373
(87) International publication number: WO 2020/225787

(56) References cited:
- CN-A- 105 784 521
- CN-U- 205 143 197
- CN-U- 207 197 985
- US-A1- 2015 212 022
- Anton Paar: "Penetrometer", , 1 January 2018 (2018-01-01), pages 1-12, XP055730867, Retrieved from the Internet: URL:https://www.anton-paar.com/?eID=docume ntsDownload&document=57229 [retrieved on 2020-09-15]

## Description

The invention relates to an analyzer of beeswax, cosmetic waxes or waxes used in pharmaceutical formulations and articles like all kinds of candles or grave lanterns, designed to discern between the natural wax and waxes modified by substances which reduce its quality, which is easy to operate, portable, able to be commonly used in case of need.

In consideration of growing prices of the natural beeswax, one of the numerous problems of the modern bee-keeping is modifying such a wax with additives. A modified wax, used by beekeepers for a low-quality foundation produces an effect of deteriorating conditions of bee families, as well as lowering the quality of other products made of it.

Among known and widely applied methods of assessing wax quality an organoleptic method is used, which, unfortunately, identifies its adulterations unsatisfactorily. Other methods, which enable unequivocal detection of adulteration of wax, is e.g. gas chromatography coupled with mass spectrometry (GCMS), FTIR spectroscopy, scanning calorimetry (DSC) or X-ray diffraction, however, such analyses are carried out in laboratories on a specialized equipment, restricting their availability to foundation makers and beekeepers. Furthermore, Anton Paar GmbH, 'Penetrometer PNR 12', 2018, pages 1-12, (URL: https://www.anton-paar.com/? eID=documentsDownload&document=57229) describes a needle penetrator device that can be used for testing wax and that comprises a holder for placing a sample with a temperature sensor, however it does not describe a heater provided above the penetrator or optocouplers for precise determination of a sample penetration time.

The object of the invention was to provide a portable device, easy to operate and able to be commonly used in case of need, serving to discern between the natural wax and a wax doped with substances which reduce its quality, by measuring physical properties discriminating them.

The wax analyzer according to the invention is constructed of a base with a display fixed on its front panel and an internal chamber, where a power unit with a microcontroller is provided to control operation of the device, to record a measurement result and send it to the display. At the upper surface of the base a holder is provided for a tested wax sample with a temperature sensor, and above it, vertically in the middle, a mechanism is installed in a form of a tractive screw connected with a stepper motor, and terminated at the bottom with the sample-penetrating element with a heater with a temperature controller arranged above it, the precise sliding movement of which is controlled by the stepper motor and stabilized by two arms of a guide with tips loosely mounted on vertical rods attached to the base. A panel with two optocouplers is also mounted on the base for determining the time of sample penetration, and the whole is enclosed in a casing with an opening window at the front panel to provide access to the holder designed for inserting the sample.

It is preferred that the element penetrating the wax sample is in a shape of a horizontally fixed rod, tube, plane such as, e.g., a knife or a vertical needle or a rod with a sharpened tip.

It is also preferred that the holder for placing a sample is located on a rotatable axis mounted on the base, which enable cutting the same sample in various places for a more detailed analysis thereof.

It is preferred that the casing is provided with open-work walls with a fan and a Peltier module with a heater, controlled by the microcontroller for faster cooling-down the interior of the analyzer to a programmed temperature to start a subsequent measurement.

An electronic circuit in a form of the properly programmed microcontroller controls the temperature of a coil which heats the element penetrating the sample within wax softening-temperature range, its pressure force on a sample, counts precisely the penetration time of the predetermined thickness of the wax depending on the kind and content of modifying components, and also stabilizes the temperature of the entire system, with the one starting a subsequent measurement.

Signals from the optocouplers determine the moment when element penetrating the sample reaches the starting and final positions in a sample. The measurements are transmitted to the microcontroller, are differentiated and compared to the penetration time range adopted for the standard e.g. the natural wax, and then displayed on an LCD screen in the form of two numerical values, one - the penetration time of the element through a tested sample and the other - the percent value expressing a difference from the analogous time for the standard wax, which illustrates presence of modifying substances.

Pure natural waxes of various origin are characterized by approximate physical features such as an elasticity or viscosity at the given temperature conditions, a melting point, and also a thermal conduction, consequently exhibiting approximate average penetration rates. Different shorter times, even by several hundred %, are exhibited by the doped waxes.

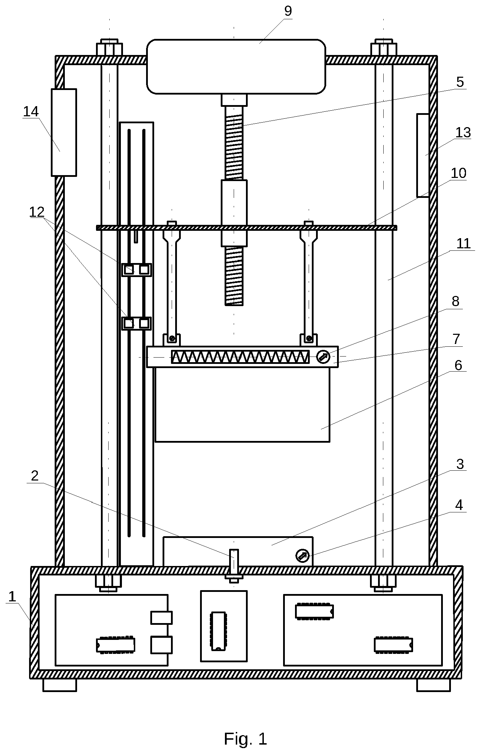

The invention is disclosed in the following embodiment and on the drawing, wherein fig. 1 illustrates the analyzer in a cross-sectional view.

The wax analyzer according to the invention is constructed of a base 1, with a display fixed on its front panel, and an internal chamber where a power unit with a microcontroller is provided. To control the mechanical part of the device, the universal, properly programmed Arduino Mega 2560 microcontroller was used, and controlled, stabilized DC power units were used for supplying power to the heater and stepper motor. At the surface of the base a holder 3 is located on a rotatable axis 2 for a tested wax sample, with a temperature sensor 4, and above it vertically in the middle, a mechanism is installed in a form of a tractive screw 5 connected with a stepper motor, and terminated at the bottom with the sample-penetrating element 6 in a form of a copper knife, a heating coil 7 and temperature sensor 8 being provided above it. Precise sliding movement of the sample penetrating element is controlled by the stepper motor 9 and stabilized by two arms of a guide 10, with loosely set tips moving along vertical rods 11 attached to the base. Also mounted on the base is a panel with two optocouplers 12 which enable determination of the sample penetration time. The whole is enclosed in a casing, with a fan 13 and a Peltier module with a heater 14 controlled by the microcontroller mounted on the internal walls thereof, and an opening window is located at the front panel to provide access to the holder designed for inserting the sample.

## Claims

1. A wax analyzer constructed of a base, with a display fixed on its front panel and an internal chamber where a power unit with a microcontroller is provided to control operation of the device, wherein at the upper surface of the base (1) a holder (3) is provided for placing a sample of the tested wax, with a temperature sensor (4), and above it vertically in the middle, a mechanism is installed which is terminated at the bottom with an element (6) penetrating the sample, **characterized in that** the mechanism is in a form of a tractive screw connected with a stepper motor, with a heater (7) being provided above the element with a temperature sensor (8), the precise sliding movement of which is controlled by the stepper motor (9) and stabilized by two arms of a guide (10) with tips movably mounted on vertical rods (11) attached to the base, on which a panel is also mounted with two optocouplers (12) which enable determination of the sample penetration time, and the whole is enclosed in a casing with an opening window at the front panel to provide access to the holder with a sample.

2. The analyzer according to claim 1, **characterized in that** the element (6) penetrating the sample is in a form of a horizontal rod, tube or plane e.g. a knife, or a vertical needle or a rod with a sharpened tip and made of a material with good heat conduction.

3. The analyzer according to claim 1, **characterized in that** the holder (3) for placing a sample is located on a rotatable axis mounted on the base (2).

4. The analyzer according to claim 1, **characterized in that** the casing is provided with open-work walls with a fan (13) and a Peltier module with a heater (14), controlled by the microcontroller.

5. The analyzer according to claim 1, **characterized in that** the casing is provided with a fan (13) and a Peltier module with a heater (14) controlled by the microcontroller mounted on the internal walls of the casing.

## Patentansprüche

1. Wachsanalysator, aufgebaut aus einer Basis mit einer an ihrer Frontplatte befestigten Anzeige und einer inneren Kammer, in der eine Stromversorgungseinheit mit einem Mikrocontroller zur Steuerung des Betriebs des Geräts vorgesehen ist, wobei an der oberen Fläche der Basis (1) ein Halter (3) zum Auflegen einer Probe des zu prüfenden Wachses vorgesehen ist, mit einem Temperatursensor (4) und darüber vertikal in der Mitte ein Mechanismus eingebaut ist, der unten mit einem die Probe durchdringenden Element (6) beendet ist, **dadurch gekennzeichnet, dass** der Mechanismus die Form einer Vorschubspindel hat, die mit einem Schrittmotor verbunden ist, wobei ein Heizung (7) über dem Element mit einem Temperatursensor (8) vorgesehen ist, dessen genaue Gleitbewegung durch den Schrittmotor (9) gesteuert wird und stabilisiert wird durch zwei Arme einer Führung (10) mit Spitzen, die beweglich an vertikalen Stangen (11) angebracht sind, die an der Basis befestigt sind, an der auch eine Platte mit zwei Optokopplern (12) angebracht ist, die eine Bestimmung der Probendurchdringungszeit ermöglichen, und das Ganze in einem Gehäuse mit einem abschließbaren Fenster in der Frontplatte eingeschlossen ist, um Zugang zum Halter mit einer Probe zu ermöglichen.

2. Analysator nach Anspruch 1, **dadurch gekennzeichnet, dass** das die Probe durchdringende Element (6) die Form eines horizontalen Stabs, Rohrs oder einer Ebene, z. B. eines Messers, oder einer senkrechten Nadel oder eines Stabs mit scharfer Spitze hat und aus einem gut wärmeleitenden Material hergestellt ist.

3. Analysator nach Anspruch 1, **dadurch gekennzeichnet, dass** der Halter (3) zum Auflegen einer Probe auf einer auf der Basis (2) gelagerten Rundachse angeordnet ist.

4. Analysator nach Anspruch 1, **dadurch gekennzeichnet, dass** das Gehäuse mit durchbrochenen Wänden mit einem Ventilator (13) und einem Peltier-Modul mit einer Heizung (14) versehen ist, gesteuert durch einen Mikrocontroller.

5. Analysator nach Anspruch 1, **dadurch gekennzeichnet, dass** das Gehäuse mit einem Ventilator (13) und einem Peltier-Modul mit einer Heizung (14) versehen ist, gesteuert durch einen an den Innenwänden des Gehäuses montierten Mikrocontroller.

## Revendications

1. Un analyseur de cire construit à partir d'une base, avec un afficheur fixé sur son panneau avant et une chambre interne où une unité d'alimentation avec un microcontrôleur est pourvue pour contrôler le fonctionnement de l'appareil, dans lequel un support (3) à la surface supérieure de la base (1) est pourvu pour placer un échantillon de la cire testée, avec un capteur de température (4), et au-dessus de la base, verticalement au milieu, un mécanisme est installé, terminé en bas par un élément (6) qui pénètre dans l'échantillon, **caractérisé en ce que** le mécanisme est sous la forme d'une vis de traction reliée à un moteur pas à pas, et un élément chauffant (7) est pourvu au-dessus de l'élément avec un capteur de température (8) dont le mouvement de coulissement précis est commandé par le moteur pas à pas (9) et stabilisé par deux bras d'une glissière (10) avec des pointes montées mobiles sur des tiges verticales (11) fixées à la base, sur laquelle un panneau est aussi monté avec deux optocoupleurs (12) pour déterminer le temps de pénétration dans l'échantillon, et le tout est placé dans un boîtier avec une fenêtre d'ouverture sur le panneau avant pour permettre l'accès au support avec un échantillon.

2. Analyseur selon la revendication 1, **caractérisé en ce que** l'élément (6) qui pénètre dans l'échantillon est sous la forme d'une tige horizontale, d'un tube ou d'un plan, par exemple un couteau, ou une aiguille verticale ou une tige avec une pointe aiguisée et faite en matériau à bonne conductivité thermique.

3. Analyseur selon la revendication 1, **caractérisé en ce que** le support (3) pour placer l'échantillon est situé sur un axe rotatif monté sur la base (2).

4. Analyseur selon la revendication 1, **caractérisé en ce que** le boîtier est muni de parois ajourées avec un ventilateur (13) et un module Peltier avec un réchauffeur (14), contrôlé par un microcontrôleur.

5. Analyseur selon la revendication 1, **caractérisé en ce que** le boîtier est muni d'un ventilateur (13) et d'un module Peltier avec un réchauffeur (14) contrôlé par le microcontrôleur monté sur les parois internes du boîtier.
